# EUROPEAN PATENT APPLICATION

(11) **EP 1 447 097 A1**
(43) Date of publication of application: **18.08.2004**
(21) Application number: 02762766.0
(22) Date of filing: 09.08.2002
(51) Int. Cl.: A61K 47/40, A61K 9/70, A61K 47/10, A61K 47/12, A61K 47/14, A61K 47/18, A61K 47/20, A61K 47/26, A61K 47/32, A61K 47/34, A61K 47/44

(54) **PERCUTANEOUS ABSORPTION TYPE MEDICINAL PREPARATION**

(30) Priority: 10.08.2001 JP 2001243792
(71) Applicant: HISAMITSU PHARMACEUTICAL CO. INC., Tosu-shi, Saga-ken 841-0017 (JP)
(72) Inventor: TERAHARA, Takaaki Hisamitsu Pharmaceutical Co.,Inc, Tsukuba-shi, Ibaraki 305-0856 (JP); CHONO, Hideharu Hisamitsu Pharmaceutical Co., Inc., Tsukuba-shi, Ibaraki 305-0856 (JP); TATEISHI, Norifumi Hisamitsu Pharmaceutical Co.Inc, Tsukuba-shi, Ibaraki 305-0856 (JP); HIGO, Naruhito Hisamitsu Pharmaceutical Co., Inc, Tsukuba-shi, Ibaraki 305-0856 (JP); SATO, Shuji, Hisamitsu Pharmaceutical Co., Inc., Tsukuba-shi, Ibaraki 305-0856 (JP)
(74) Representative: Ede, Eric
(86) International application number: PCT/JP2002/008164
(87) International publication number: WO 2003/013613

(57) **Abstract**

A percutaneous absorption type medical preparation which, even when the base is nonaqueous, attains sufficient percutaneous absorbability and which is reduced in skin irritation and has improved safety. It is characterized by containing one or more selected from the group consisting of cyclodextrin and cyclodextrin derivatives and one or more drugs.

## Description

### [Detailed Description of the Invention]

### [Technical Field]

The invention relates to a percutaneous absorption type medical preparation comprising cyclodextrin and/or cyclodextrin derivatives and a drug.

### [Background Art]

As administration methods for drugs, an oral administration using tablets, capsules, syrups or the like has been made in many drugs. However, in case of oral administration, there were drawbacks that a drug was susceptible to a first-pass effect in the liver after absorption of the drug and an unnecessarily high blood concentration was recognized for a while after the administration, whereby a side effect occurs easily and so on. In order to eliminate such drawbacks of oral administration, development for percutaneous absorption type medical preparations has actively been carried out. The percutaneous absorption type medical preparations cover not only those drawbacks, but are expected to have merits such as reduction of administration frequency, improvement of compliance and simplicity of administration and its stoppage, and are known to be useful particularly in aged and infant patients.

However, since the normal skin has a barrier function to protect the penetration of foreign substances into the body, a sufficient percutaneous absorption of a blended pharmaceutically effective ingredient is hardly attained in many cases. Also, since the stratum corneum having a barrier capacity is high in lipophilicity, generally the permeability of a drug is extremely low.

Therefore, a device to increase the percutaneous absorbability through a stratum corneum of the skin is needed, and so an absorption accelerating composition containing various percutaneous absorption promoters and a complex formation of polymer, which uses a solid dispersoid, and a drug has been investigated. Among those, cyclodextrin is known to improve a drug solubility by forming a complex with a drug, and it is known also in the field of external preparations to accelerate a percutaneous absorption of a drug by containing cyclodextrin as a component. In particular, it is known that cyclodextrin is excellent in improving the solubility of a drug in water, and the effect in a base containing water is expected (Yakugaku Zasshi, 101, 857-873, 1981). Further, a method to strengthen the effect of a absorption promoter by forming a complex with a percutaneous absorption promoter and cyclodextrin is reported.

In the meantime, hydroxypropyl-β-cyclodextrin (HP-β-CyD), which is a cyclodextrin derivative, is a substance in which 2-hydroxypropyl group is introduced to 2 and 6 positions of natural type β-CyD, and has characteristics of high solubility in water and lower alcohols and an amphiphilic property compared with the natural type, and it is a derivative type cyclodextrin characterized in that it is no crystalline and is amorphous. As for HP-β-CyD having such characteristics, it is reported that it is useful for a drug having low water solubility and improves the dissolution rate in water by improving solubility of the drug (WO85/02767).

However, in the known art as above, what are obtained are limited to solubility improvement as well as a percutaneous absorption accelerating effect for a drug in a percutaneous absorption type medical preparation with an aqueous base, and an effect on a nonaqueous base is not disclosed. Although a nonaqueous matrix type adhesive preparation with a nonaqueous base is generally used as a dosage form of a systemic percutaneous absorption type medical preparation, an absorption promoter, which has a sufficient percutaneous absorption effect for a drug and is low in the skin irritation, has not been obtained yet.

### [Disclosure of the Invention]

Accordingly, the problem of the invention is to provide a percutaneous absorption type medical preparation which, even when the base is nonaqueous, attains sufficient percutaneous absorbability and which is reduced in skin irritation and has improved safety.

The inventors made extensive researches to solve such problems and found that a percutaneous absorption type medical preparation containing cyclodextrin or a cyclodextrin derivative with a drug solves the above problems and accomplished the invention.

Namely, the invention relates to a percutaneous absorption type medical preparation containing one or more members selected from the group consisting of cyclodextrin and cyclodextrin derivatives and one or more drugs in a nonaqueous base.

Further, the invention relates to the above-mentioned percutaneous absorption type medical preparation characterized in that it is a nonaqueous matrix type adhesive preparation.

Also, the invention relates to the above-mentioned percutaneous absorption type medical preparation characterized in that the molar ratio of cyclodextrin and/or a cyclodextrin derivative to a drug is 1:0.1-1:10.

Further, the invention relates to the above-mentioned percutaneous absorption type medical preparation characterized in that cyclodextrin and the cyclodextrin derivative are β-cyclodextrin and a β-cyclodextrin derivative.

Also, the invention relates to the above-mentioned percutaneous absorption type medical preparation characterized in that the β-cyclodextrin derivative is hydroxypropyl β-cyclodextrin.

Further, the invention relates to the above-mentioned percutaneous absorption type medical preparation characterized in that the drug is a drug containing a basic functional group or chemically acceptable salts thereof.

Also, the invention relates to the above-mentioned percutaneous absorption type medical preparation characterized in that the drug is procaterol or chemically acceptable salts thereof, containing β-cyclodextrin.

Further, the invention relates to the above-mentioned percutaneous absorption type medical preparation characterized in that the drug is pergolide or chemically acceptable salts thereof and the preparation contains β-cyclodextrin.

Also, the invention relates to the above-mentioned percutaneous absorption type medical preparation characterized in that the base contains one or more members selected from the group consisting of polyisoprene, polystyrene, polyethylene, polybutadiene, styrene-butadiene copolymer, styrene-isoprene-styrene block copolymer, styrene-butylene-styrene block copolymer, butyl rubber, natural rubber, styrene-butadiene-styrene block copolymer, polysiloxane and an acrylic type polymer.

Further, the invention relates to the above-mentioned percutaneous absorption type medical preparation characterized in that the base contains styrene-isoprene-styrene block copolymer and/or polyisobutylene.

Also, the invention relates to the above-mentioned percutaneous absorption type medical preparation characterized in that the acrylic type polymer contains at least one member of acrylates.

Further, the invention relates to the above-mentioned percutaneous absorption type medical preparation characterized in that the acrylates are 2ethylhexyl acrylate and/or butyl acrylate.

Also, the invention relates to the above-mentioned percutaneous absorption type medical preparation characterized in that the preparation comprises a plasticizer.

Further, the invention relates to the above-mentioned percutaneous absorption type medical preparation characterized in that the plasticizer contains one or more members selected from the group consisting of mineral oils, vegetable oils and fatty acid esters of C₂-C₂₀ fatty acids and C₂-C₂₀ fatty alcohols.

Also, the invention relates to the above-mentioned percutaneous absorption type medical preparation characterized in that the plasticizer comprises one or more selected from the group consisting of liquid paraffin, polybutene, isopropyl myristate, diethyl sebacate and hexyl laurate.

Further, the invention relates to the above-mentioned percutaneous absorption type medical preparation characterized in that the preparation comprises an absorption promoter.

Also, the invention relates to the above-mentioned percutaneous absorption type medical preparation characterized in that the absorption promoter is one or more members selected from the group consisting of lauric acid diethanolamide, sorbitan monolaurate, glycerol monolaurate, glycerol monooleate, glycerol monocaprate, glycerol monocaprylate, polyoxyethylene (4) lauryl ether and pyrothiodecane.

Further, the invention relates to the above-mentioned percutaneous absorption type medical preparation characterized in that the absorption promoter contains lauric acid diethanolamide and/or sorbitan monolaurate.

Also, the invention relates to the above-mentioned percutaneous absorption type medical preparation comprising an organic acid and/or chemically acceptable salts thereof.

Further, the invention relates to the above-mentioned percutaneous absorption type medical preparation characterized in that the organic acid is one or more members selected from the group consisting of acetic acid, propionic acid, lactic acid and salicylic acid.

Also, the invention relates to the above-mentioned percutaneous absorption type medical preparation characterized in that the organic acid is acetic acid.

Also, the invention relates to the above-mentioned percutaneous absorption type medical preparation characterized in that it is obtained dissolving or dispersing a mixture containing cyclodextrin and/or the cyclodextrin derivative with the drug in the nonaqueous base.

Further, the invention relates to the above-mentioned percutaneous absorption type medical preparation wherein the preparation is obtained by dissolving the mixture in a solvent, followed by evaporating the solvent to dryness.

Also, the invention relates to the above-mentioned percutaneous absorption type medical preparation characterized in that it is obtained by grinding the mixture in a dry or wet condition in the same batch.

Further, the invention relates to the above-mentioned percutaneous absorption type medical preparation characterized in that the mixture further comprises a plasticizer.

Also, the invention relates to the above-mentioned percutaneous absorption type medical preparation characterized in that the mixture further comprises an absorption promoter.

The percutaneous absorption type medical preparation of the invention even in a nonaqueous base can dissolve drugs in the base, and is excellent not only in the skin permeability but also in the skin irritation and content stability, or in the physical stability of the base.

Also, by addition of the organic acid to the percutaneous absorption type medical preparation of the invention, the effects improve further, and a surprisingly high percutaneous absorption of drugs is obtained.

Further, the percutaneous absorption type medical preparation of the invention is obtained by dry grinding or wet grinding of a mixture containing cyclodextrin and/or a cyclodextrin derivative with a drug and by dissolving or dispersing the mixture in a base of the percutaneous absorption type medical preparation, whereby the percutaneous absorption of the drug can extremely be increased. Also, in case of adding the plasticizer, if needed, the organic acid or its salt, or the absorption promoter, are added beforehand to a mixture containing cyclodextrin and/or a cyclodextrin derivative with a drug, followed by dry grinding or wet grinding of the mixture to be able to give a higher percutaneous absorption effect.

### [Brief Description of Drawing]

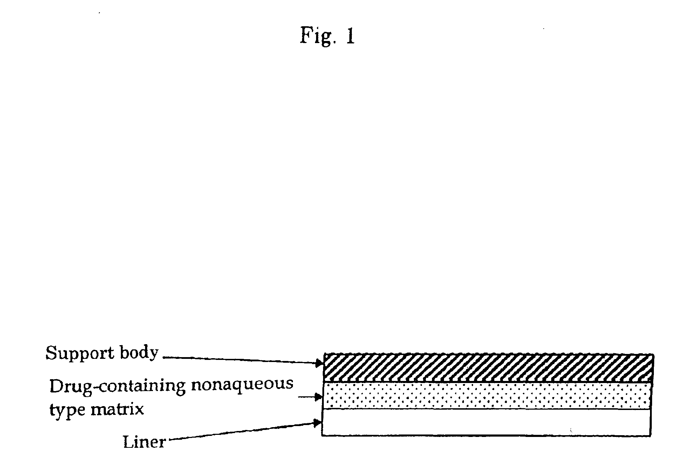

Fig. 1 is an example of the constitution of a nonaqueous matrix type adhesive preparation of the invention.

### [Mode for carrying out the Invention]

The composition and form in an adhesive layer are explained exempli fying a nonaqueous matrix type adhesive preparation which is one embodiment of the percutaneous absorption type medical preparations of the invention.

The nonaqueous matrix type adhesive preparation of the invention has a form consisting of an adhesive layer, containing polymer showing an adhesive property and a drug, and a support body at the back of the adhesive layer as shown in Fig. 1.

As a drug used in the adhesive layer of the nonaqueous matrix type adhesive preparations of the invention, there is no particular limitation on its member if it is a percutaneously absorbable drug, and examples include hypnotic-sedative agents (fluazepam hydrochloride, rilmazafone hydrochloride, etc.), anti-inflammatory agents (butorphanol tartarate, persoxal citrate, etc.), excitation-analeptic agents (methamphetamine hydrochloride, methylphenidate hydrochloride, etc.), psychotropic agents (sertraline hydrochloride, fluvoxamine maleate, etc.), local anesthetic agents (lidocain hydrochloride, procaine hydrochloride), agents for urinary organs (oxybutynin hydrochloride, etc.), skeletal muscle relaxants (tizanidine hydrochloride, eperisone hydrochloride, pridinol mesilate, etc.), autonomic agents (carpronium chloride, neostigmine bromide, etc.), anti-Parkinson's disease agents (pergolide mesylate, bromocriptine mesylate, trihexyphenidyl hydrochloride, amantadine hydrochloride, etc.), antihistaminic agents (clemastine fumarate, diphenhydramine tannate, etc.), bronchodilator agents (tulobuterol hydrochloride, procaterol hydrochloride, etc.), cardiotonic agents (isoprenaline hydrochloride, dopamine hydrochloride, etc.), coronary dilators (diltiazem hydrochloride, verapamil hydrochloride, etc.), peripheral vasodilators (nicametate citrate, tolazoline hydrochloride, etc.), cardiovascular agents (flunarizine hydrochloride, nicardipine hydrochloride benizipine hydrochloride, efonizipine chloride, etc.), antiarrhythmic agents (propranolol hydrochloride, alprenolol hydrochloride, etc.), antiallergic agents (ketotifen fumarate, azelastine hydrochloride, etc.), anti-dizziness agents (betahistine mesilate, difenidol hydrochloride, etc.), serotonin receptor antagonistic antiemetics (ondansetron hydrochloride, etc.), narcotic analgesic agents (morphine sulfate, fentanyl citrate, etc.), non-steroidal anti-infammatory agents (ketoprofen, diclofenac sodium, etc.), antibiotics (erythromycin, chloramphenicol, etc.), sex hormons (progesterone, estradiol, etc.) and coronary vasodilators (nitroglycerin, isosorbide dinitrate, etc.).

Further, these drugs may be used alone or in a combination of two or more of them, and either form of drug, an inorganic salt or an organic salt, may naturally be comprised. Also, considering a sufficient permeable amount as the adhesive preparations and irritation to the skin such as rubor, drugs can be blended in an amount, for example, 0. 1-50 mass% based on the mass of the total composition in the adhesive layer.

As polymers to be contained in the base of the nonaqueous matrix type adhesive preparation of the invention, an acrylic type polymer or a rubber polymer can be used.

As the acrylic type polymer there is no particular restriction if it is a copolymerized substance containing at least one of (meth)acrylic acid derivatives represented by 2-ethylhexyl acrylate, methyl acrylate, butyl acrylate, hydroxyethyl acrylate, 2-ethylhexyl methacrylate and the like, though adhesive agents such as acrylic acid·octyl acrylate copolymer, acrylate·vinyl acetate copolymer, 2-ethylhexyl acrylate·vinylpyrrolidone copolymer, 2-ethylhexyl acrylate·2-ethylhexyl methacrylate·dodecyl methacrylate copolymer, methyl acrylate·2-ethylhexyl acrylate copolymer, resin emulsion and acrylic type polymer contained in acrylic resin alkanolamine liquid, which are, for example, published in Drug Additives Encyclopedia 2000 (edited by Japan Drug Additives Society) as adhesive agents, DURO-TAK acrylic adhesive agent series (manufactured by National Starch and Chemicals Co., Ltd.), Eudragit series (Higuchi Syoukai) and the like, can be used.

As the rubber polymer, illustrative are styrene-isoprene-styrene block copolymer (hereinafter abbreviated as SIS), isoprene rubber, polyisobutylene (hereinafter abbreviated as PIB), styrene-butadiene-styrene block copolymer (hereinafter abbreviated as SBS), styrene-butadiene rubber (hereinafter abbreviated as SBR), polysiloxane and the like. Among them SIS and PIB are preferable, and SIS is in particular preferable.

The hydrophobic polymer may be used in a mixture of two or more members, and considering formation of the adhesive layer and sufficient permeability, the blending amount of these polymers based on the mass of the total composition may be preferably 10-90 mass%, more preferably 30-90 mass%, in particular preferably 30-70 mass%.

Cyclodextrin derivatives used in the invention mean those in which cyclodextrin is derivatized, for example, by etherification and the like considering solubility, skin safety and the like of a complex with a drug, and any one may be used if it attains the object of the invention.

The substitutional degree of the cyclodextrin derivatives is preferably that of 1-10, for example, in case of the etherification substitution by a alkyl group and the like.

Also, cyclodextrin or cyclodextrin derivatives used in the invention can be any cyclodextrin of α-cyclodextrin, β-cyclodextrin or γ-cyclodextrin, which can be selected and used considering a drug used, solubility of a complex with the drug and the like, though particularly β-cyclodextrin is preferable, and so are hydroxypropyl-β-cyclodextrin substituted at 2 position and 6 position of β-cyclodextrin with 2-hydroxypropyl group. Further, in case of using procaterol or salts thereof as a drug, complexes with β-cyclodextrin are preferable from the view points of high skin absorbability and reduction of skin irritation, and in case of using pergolide or salts thereof as a drug, complexes with hydroxypropyl-β-cyclodextrin are preferable.

The blending ratio (drug:cyclodextrin, etc.) of the cyclodextrinderivatives and cyclodextrin to a drug is preferably 1:0.1-1:10.

In the invention it is desired to have an organic acid be contained further in the adhesive layer, and as organic acids used, illustrative are aliphatic (mono-, di-, tri-)carboxylic acids (e.g., acetic acid, propionic acid, iso-butylic acid, caproic acid, caprylic acid, lactic acid, maleic acid, pyruvic acid, oxalic acid, succinic acid, tartaric acid, etc.), aromatic carboxylic acids (e.g., phthalic acid, salicylic acid, bezoic acid, acetyl salicylic acid, etc.), alkyl sulfonic acids (e.g., methane sulfonic acid, ethane sulfonic acid, propyl sulfonic acid, butane sulfonic acid, polyoxyethylene alkyl ether sulfonic acid, etc.), alkyl sulfonic acid derivatives (e.g., N-2-hydroxyethyl-piperidine-N'-2-ethane sulfonic acid (hereinafter abbreviated as HEPES), etc.) and cholic acid (e. g. , dehydrocholic acid, etc.). Among them acetic acid, propionic acid, lactic acid and salicylic acid are preferable, and acetic acid is in particular preferable. Also, in these organic acids, salts thereof or a mixture with a salt may be used.

Considering a sufficient permeation amount as the adhesive preparation and the irritation to the skin, these organic acids can be blended preferably in the amount of 0.01-20 mass% based on the mass of the total composition of the adhesive layer, more preferably 0.1-15 mass%, in particular preferably 0.1-10 mass%.

An absorption promoter may be contained in an adhesive layer of the adhesive preparations of the invention, and as the absorption promoter which can be used, any compound in which an absorption promoting effect in the skin has been so far shown maybe used. Examples includes C₆-C₂₀ fatty acids, fatty alcohols, fatty acid esters, amides or ethers, aromatic organic acids, aromatic alcohols, aromatic fatty acid esters or ethers (these may be saturated or unsaturated, and may be either cyclic, straight or branched), furthermore lactates, acetates, monoterpene compounds, sesquiterpene compounds, Azone, Azone derivatives, pyrothiodecane, glycerol fatty acid esters, propylene glycol fatty acid esters, sorbitan fatty acid esters (Span type), polysorbates (Tween type), polyethylene glycol fatty acid esters, polyoxyethylene hardened castor oils (HCO type), polyoxyethylene alkyl ethers, sucrose fatty acid esters, vegetable oils and the like.

Specifically, caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methy laurate, hexyl laurate, lauric acid diethanolamide, isopropyl myristate, myristyl myristate, octyldecyl myristate, cetyl palmitate, salicylic acid, methyl salicylate, ethylene glycol salicylate, cinnamic acid, methyl cinnamate,cresol,cetyllactate,lauryllactate, ethyl acetate, propyl acetate, geraniol, thymol, eugenol, terpineol, 1-menthol, borneol, d-limonene, isoeugenol, isoborneol, nerol, dl-camphor, glycerol monocaprylate, glycerol monocaprate, glycerol monolaurate, glycerol monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pyrothiodecane and olive oil are preferred, and lauryl alcohol, isostearyl alcohol, lauric acid diethanolamide, glycerol monocaprylate, glycerol monocaprate, glycerol monooleate, sorbitan monolaurate, propylene glycol monolaurate, polyoxyethylene lauryl ether and pyrothiodecane are particularly preferred.

The absorption promoter may be used in a mixture of two or more members, and considering sufficient permeability as the adhesive preparation and irritation to the skin such as rubor, edema and the like, it can be blended preferably in 0.01-20 mass% based on the mass of the total composition of the adhesive layer, more preferably 0.05-10 mass%, in particular preferably 0.1-5 mass%.

A plasticizer may be contained in the adhesive layer of the preparations of the invention, and the plasticizer can be used as solvent of a wet grinding. As usable plasticizers, illustrative are petroleum oils (e.g., paraffin type process oil, naphthalene type process oil, aromatic type process oil, etc.), squalane, squalene, vegetable oils (e.g., olive oil, camellia oil, castor oil, tall oil, peanut oil), silicone oil, dibasic acid esters (e.g., dibutylphthalate, dioctylphthalate, etc.), liquefied rubber (e.g., polybutene, liquefied isoprene rubber), liquefiedfattyacidesters (isopropylmyristate, hexyl laurate, diethyl sebacate, diisopropyl sebacate), diethylene glycol, polyethylene glycol, glycol salicylate, propylene glycol, dipropylene glycol, triacetin, triethyl citrate, crotamiton and the like. In particular, liquid paraffin, liquefied polybutene, crotamiton, diethyl sebacate and hexyl laurate are preferred.

These constituents may be used in a mixture of two or more members, and considering sufficient permeability and maintenance of an sufficient agglutinative strength as the adhesive preparations, the blending amount of such plasticizers based on the total composition in the adhesive layer can be preferably 10-70 mass% in total, more preferably 10-60 mass%, in particular preferably 10-50 mass%.

A tackifying resin may be contained in an adhesive layer of the adhesive preparations of the invention, and as usable tackifying resins, illustrative are rosin derivatives (e.g., rosin, glycerol esters of rosin, hydrogenated rosin, glycerol esters of hydrogenated rosin, pentaerythritol esters of rosin, etc.), alicyclic saturated hydrocarbon resins (e.g., Arcon P 100, manufactured by Arakawa Kagaku Kogyo Co., Ltd.), aliphatic hydrocarbon resins (e.g., Quintone B 170, manufactured by Nihon Zeon Co., Ltd.), terpene resins (e.g., Clearon P-125, manufactured by Yasuhara Chemicals Co., Ltd.), maleic acid resins and the like. In particular, glycerol esters of hydrogenated rosin, alicyclic saturated hydrocarbon resins, aliphatic hydrocarbon resins and terpene resins are preferred.

Considering an enough adhesive strength as the adhesive preparations and the irritation to the skin at the time of dissection, the blend amount of such tackifying resins based on the total composition in the adhesive layer can be preferably 5-70 mass%, more preferably 5-60 mass%, in particular preferably 10-50 mass%.

Also, if required, antioxidants, fillers, cross-linking agents, preservatives or UV absorbers can be used. As antioxidants, tocopherol and its ester derivatives, ascorbic acid, ascorbic acid-stearic acid ester, nordihydroguaretic acid, dibutyl hydroxy toluene (BHT), butyl hyroxy anisole and the like are desirable. As fillers, calcium carbonate, magnesium carbonate, silicate (e.g., aluminum silicate, magnesium silicate, etc.), silicic acid, barium sulfate, calcium sulfate, calcium zincate, zinc oxide, titanic oxide and the like are desirable. As cross-linking agents, thermosetting resins such as amino resins, phenol resins, epoxy resins, alkyd resins and unsaturated polyesters, isocyanate compounds, block isocyanate compounds, organic type cross-linking agents, and inorganic type cross-linking agents such as metals or metal compounds, are desirable. As preservatives, ethyl p-hydroxy benzoate, propyl p-hydroxy benzoate, butyl p-hydroxy benzoate and the like are desirable. As UV absorbers, p-amino benzoic acid derivatives, anthranilic acid derivatives, salicylic acid derivatives, coumarin derivatives, amino acid type compounds, imidazoline derivatives, pyrimidine derivatives, dioxane derivatives and the like are desirable.

Such antioxidants, fillers, cross-linking agents, preservatives and UV absorbers can be blended preferably in an amount of not more than 10 mass% in total based on the mass of the total composition in the adhesive layer of the adhesive preparations, more preferably not more than 5 mass% and in particular preferably not more than 2 mass%.

The drug-containing adhesive layer containing the composition described above can be prepared by any method. For example, a base composition containing a drug is heat-melted, coated on removable paper or a support body, followed by affixing to the support or the removable paper to give the present preparations. Also, base ingredients containing a drug are dissolved in solvent such as toluene, hexane or ethyl acetate, spread on removable paper or a support body, dried to remove solvent, followed by affixing it to the support or the removable paper to give the present preparations.

Preparation of the adhesive layer can be made, for example, mixing cyclodextrin or the like with a drug, dissolving in solvent, and then evaporating the solvent to dryness to give a mixture, further dissolving or dispersing the mixture in the base of a nonaqueous matrix type adhesive preparation. The above mixture is ground under dry or wet conditions in the same batch, and a complex becomes amorphous, preferably resulting to improvement of the solubility. Further, in case of adding the plasticizer, the absorption promoter, the organic acid or the like, it is preferable for the same reasons as described above that these are added beforehand to a mixture containing cyclodextrin and the like with a drug, and then the mixture is ground under dry or wet conditions in the same batch, followed by dissolving or dispersing it in the base of a nonaqueous matrix type adhesive preparation.

For example, an adhesive preparation of the invention can consist of the above adhesive layer, in addition, a support layer to support it, and a removable-paper layer set on the adhesive layer.

As to the support layer, an elastic or a non-elastic support body can be used, and for example, it can be selected from fabric, polyurethane, polyester, polyvinyl acetate, polyvinylidene chloride, polyethylene, polyethylene terephthalate, aluminum sheet and the like, or composite materials thereof.

Also, if the nonaqueous matrix type adhesive preparation of the invention is a nonaqueous composition containing cyclodextrin or a cyclodextrin derivative with a drug, the other constructions or materials of each constituent may be of any kind.

### [Example]

In the following, the invention is explained in more detail by the examples. The invention, however, is not limited to these examples, and various changes may be made without departing from the spirit of the invention. Further, in the examples, all % mean % by mass.

### (Example 1)

| | |
|---|---|
| SIS | 20.0% |
| PIB | 10.0% |
| Hydrogenated rosin ester | 34.0% |
| Liquid paraffin | 20.0% |
| Sodium acetate | 3.0% |
| Acetic acid | 2.0% |
| β-CyD | 5.0% |
| Lauric acid diethanolamide | 3.0% |
| Procaterol hydrochloride | 3.0% |
| Total amount | 100.0% |

Procaterol hydrochloride, β-CyD, liquid paraffin, lauric acid diethanolamide, sodium acetate and acetic acid were beforehand ground using a grinder of wet process and mixed with the remaining ingredients dissolved in toluene. After the mixture was coated on removable paper, the solvent was removed by drying, followed by affixing it to a support body to give a matrix adhesive preparation of the invention.

### (Example 2)

| | |
|---|---|
| SIS | 10.0% |
| Acrylic polymer | 20.0% |
| (Duro-Tak87-4098, National Starch and Chemicals Co., Ltd.) Alicyclic saturated hydrocarbon resin (Arcon P100) | 30.0% |
| Sodium acetate | 1.0% |
| Acetic acid | 3.0% |
| Liquid paraffin | 20.0% |
| β-CyD | 7.0% |
| Sorbitan monolaurate | 5.0% |
| Procaterol hydrochloride | 4.0% |
| Total amount | 100.0% |

Procaterol hydrochloride, β-CyD, Sorbitan monolaurate, liquid paraffin, sodium acetate and acetic acid were beforehand ground using a grinder of wet process and then mixed with the remaining ingredients dissolved in a mixed solvent of toluene and ethyl acetate. After the mixture was coated on removable paper, the solvent was removed by drying, followed by affixing it to a support body to give a matrix adhesive preparation of the invention.

### (Example 3)

| | |
|---|---|
| SIS | 10.0% |
| Acrylic polymer | 16.0% |
| (Duro-Tak87-4098, National Starch and Chemicals Co., Ltd.) Alicyclic saturated hydrocarbon resin (Arcon P100) | 32.0% |
| Liquid paraffin | 16.0% |
| Lauryl alcohol | 3.0% |
| Lauric acid diethanolamide | 5.0% |
| Acetic acid | 4.0% |
| β-CyD | 10.0% |
| Procaterol | 4.0% |
| Total amount | 100.0% |

Procaterol, β-CyD, lauryl alcohol, lauric acid diethanolamide, acetic acid and liquid paraffin were beforehand put in a mortar and mixed thoroughly, followed by mixing with the remaining ingredients dissolved in amixed solvent of toluene and ethyl acetate. After the mixture was coated on removable paper, the solvent was removed by drying, followed by affixing it to a support body to give a matrix adhesive preparation of the invention.

### (Example 4)

| | |
|---|---|
| SIS | 15.0% |
| Acrylic polymer | 14.0% |
| (TSR, Sekisui Kagaku Kogyou) | |
| Hydrogenated rosin ester | 25.0% |
| Liquid paraffin | 22.0% |
| HP-β-CyD | 10.0% |
| Sodium acetate | 2.0% |
| Lauric acid diethanolamide | 3.0% |
| Pergolide mesylate | 9.0% |
| Total amount | 100.0% |

Pergolide mesylate, HP-β-CyD, sodium acetate, lauric acid diethanolamide and liquid paraffin were beforehand ground using a grinder of wet process and then mixed with the remaining ingredients dissolved in a mixed solvent of hexane and ethyl acetate. After the mixture was coated on removable paper, the solvent was removedby drying, followedbyaffixing it to a support body to give a matrix adhesive preparation of the invention.

### (Example 5)

| | |
|---|---|
| SIS | 10.0% |
| Acrylic polymer | 20.0% |
| (Duro-Tak87-4098, National Starch and Chemicals Co., Ltd.) Alicyclic saturated hydrocarbon resin (Arcon P100) | 30.0% |
| Sodium acetate | 1.0% |
| Acetic acid | 3.0% |
| Liquid paraffin | 18.0% |
| HP-β-CyD | 7.0% |
| Sorbitan monolaurate | 5.0% |
| Pergolide mesylate | 6.0% |
| Total amount | 100.0% |

Pergolide mesylate, HP-β-CyD, sorbitan monolaurate, liquid paraffin and sodium acetate were beforehand ground using a grinder of wet process and then mixed with the remaining ingredients dissolved in a mixed solvent of toluene and ethyl acetate. After the mixture was coated on removable paper, the solvent was removedbydrying, followedbyaffixing it to a support body to give a matrix adhesive preparation of the invention.

### (Examples 6)

Except using α-CyD instead of β-CyD, the ingredients and the experimental processes were identical to those in Example 2.

### (Examples 7)

Except using α-CyD instead of β-CyD, the ingredients and the experimental processes were identical to those in Example 3.

### (Examples 8)

Except using α-CyD instead of HP-β-CyD, the ingredients and the experimental processes were identical to those in Example 5.

### [Comparative Example]

### (Comparative example 1)

Except not containing β-CyD, the ingredients and the experimental processes were identical to those in Example 1.

### (Comparative example 2)

Except not containing β-CyD, the ingredients and the experimental processes were identical to those in Example 2.

### (Comparative example 3)

Except not containing β-CyD, the ingredients and the experimental processes were identical to those in Example 3.

### (Comparative example 4)

Except not containing HP-β-CyD, the other ingredients and the experimental processes were identical to those in Example 4.

### (Comparative example 5)

Except not containing HP-β-CyD, the other ingredients and the experimental processes were identical to those in Example 4.

### (Skin permeability test in hairless mice)

A back part skin of a hairless mouse was stripped, and the dermal side was placed to a receptor layer side and installed in a flow-through cell (5 cm²) in which warm water of 37°C was circulated around the outer part. Each of the adhesive preparations obtained in the examples 1-8 as well as the comparative examples 1-5 was coated on the stratum corneum side, and samplings were carried out at every one hour for 18 hours at a rate of 5 ml/hour (hr) using the physiological saline in the receptor layer. As to the receptor solutions obtained at every hour, the flow amounts were accurately measured, and the drug concentrations were measured by a high-performance liquid chromatography, followed by the calculation of the permeation rate per hour to determine the skin permeation velocity per unit area at the steady state. The results are shown in Tables 1 and 2.

### (Primary skin irritation test in rabbits)

The primary skin irritation of the preparations obtained in the examples 1-5 was tested with Draizemethod. The PII values obtained in each preparation are shown in Table 1.

### (Physical property test for preparations)

As to the preparations obtained in the examples 1-5, the adhesive strength was measured by a probe tack tester and a peel tester, and the agglutinative strength by using a creep tester. As the results, those having no problem in the physical properties were evaluated as o and those having a problem were evaluated as X. The results are shown in Table 1.

**Table 1**

| | Skin permeation rate (µg/cm²/hr) | PII values | Physical properties of preparations |
|---|---|---|---|
| Example 1 | 2.5 | 0.5 | ○ |
| Example 2 | 1.8 | 0.4 | ○ |
| Example 3 | 3.2 | 0.8 | ○ |
| Example 4 | 6.0 | 1.0 | ○ |
| Example 5 | 2.5 | 0.7 | ○ |

**Table 2**

| | Skin permeation Rate (µg/cm²/hr) |
|---|---|
| Example 6 | 0.4 |
| Example 7 | 0.5 |
| Example 8 | 1.4 |
| Comparative example 1 | 0.2 |
| Comparative example 2 | 0.1 0.1 |
| Comparative example 3 | 0.3 0.3 |
| Comparative example 4 | 2.2 2.2 |
| Comparative example 5 | 1.2 |

As is evident from the results shown in Tables 1 and 2, it was found that the nonaqueous matrix type adhesive preparations obtained in each example of the invention were high in the skin permeation rate of the drugs compared with the preparations obtained in each Comparative example, and that the preparations of the Examples could sufficiently be up to practical use also in skin irritation and physical properties of the preparations. Further, it was recognized that the complex with β-cyclodextrin in case of using procaterol or its salt as well as the complex with the β-cyclodextrin derivative in case of using pergolide or its salt, are high in particular in skin permeability, and that solubility, stability and like of the complexes are different according to the drugs and the cyclodextrins used. Therefore, it was recognized that by selection of an appropriate cyclodextrin according to the aim and by formation of a complex, its physical properties an be changed.

### [Industrial Applicability]

Since a sufficient skin permeability of drugs has been obtained according to the percutaneous absorption type medical preparation of the invention, the drugs can be efficiently absorbed into the blood, circulating via the skin. Also, in the systemic nonaqueous matrix type adhesive preparations, the sufficient effects have been obtained, and therefore side effects of the gastrointestinal system observed in case of an oral administration, and also side effects of the central nervous system which can occur due to a rapid increase of the blood concentration can be avoided. Further, they are particularly effective as external preparations aiming at the percutaneous application due to the percutaneous absorption effect as well as the low irritation to the skin.

## Claims

1. (as amended) A percutaneous absorption type medical preparation comprising in a nonaqueous base one or more selected from the group consisting of cyclodextrin and cyclodextrin derivatives, one or more drugs, an organic acid and/or chemically acceptable salts thereof.

2. The percutaneous absorption type medical preparation according to claim 1 **characterized in that** it is a nonaqueous matrix type adhesive preparation.

3. The percutaneous absorption type medical preparation according to claim 1 **characterized in that** the molar ratio of cyclodextrin and/or a cyclodextrin derivative to a drug is 1:0.1-1:10.

4. The percutaneous absorption type medical preparation according to claim 1 **characterized in that** cyclodextrin and the cyclodextrin derivative are β-cyclodextrin and a β-cyclodextrin derivative.

5. The percutaneous absorption type medical preparation according to claim 4 **characterized in that** the β-cyclodextrin derivative is hydroxypropyl β-cyclodextrin.

6. The percutaneous absorption type medical preparation according to claim 1 **characterized in that** the drug is a drug containing a basic functional group or chemically acceptable salts thereof.

7. The percutaneous absorption type medical preparation according to claim 6 **characterized in that** the drug is procaterol or chemically acceptable salts thereof and the preparation contains β-cyclodextrin.

8. The percutaneous absorption type medical preparation according to claim 6 **characterized in that** the drug is pergolide or chemically acceptable salts thereof, containing β-cyclodextrin.

9. The percutaneous absorption type medical preparation according to claim 1 **characterized in that** the base contains one or more selected from the group consisting of polyisoprene, polystyrene, polyethylene, polybutadiene, styrene-butadiene copolymer, styrene-isoprene-styrene block copolymer, styrene-butylene-styrene block copolymer, butyl rubber, natural rubber, styrene-butadiene-styrene block copolymer, polysiloxane and an acrylic type polymer.

10. The percutaneous absorption type medical preparation according to claim 9 **characterized in that** the base contains styrene-isoprene-styrene block copolymer and/or polyisobutylene.

11. The percutaneous absorption type medical preparation according to claim 9 **characterized in that** the acrylic type polymer contains at least one acrylates.

12. (as amended) The percutaneous absorption type medical preparation according to claim 11 **characterized in that** the acrylates are 2-ethylhexyl acrylate and/or butyl acrylate.

13. The percutaneous absorption type medical preparation according to claim 1 **characterized in that** the preparation comprises a plasticizer.

14. The percutaneous absorption type medical preparation according to claim 13 **characterized in that** the plastcizer contains one or more selected from the group consiting of mineral oils, vegetable oils and fatty acid esters of C₂-C₂₀ fatty acids and C₂-C₂₀ fatty alcohols.

15. The percutaneous absorption type medical preparation according to claim 13 **characterized in that** the plasticizer comprises one or more selected from the group consiting of liquid paraffin, polybutene, isopropylmyristate, diethyl sebacate and hexyl laurate.

16. The percutaneous absorption type medical preparation according to claim 1 **characterized in that** the preparation comprises an absorption promoter.

17. The percutaneous absorption type medical preparation according to claim 16 **characterized in that** the absorption promoter is one or more selected from the group consisting of lauric acid diethanolamide, sorbitan monolaurate, glycerol monolaurate, glycerol monooleate, glycerol monocaprate, glycerol monocaprylate, polyoxyethylene (4) lauryl ether and pyrothiodecane.

18. The percutaneous absorption type medical preparation according to claim 16 **characterized in that** the absorption promoter contains lauric acid diethanolamide and/or sorbitan monolaurate.

19. (as deleted)

20. (as amended) The percutaneous absorption type medical preparation according to claim 1 **characterized in that** the organic acid is one or more selected from the group consisting of acetic acid, propionic acid, lactic acid and salicylic acid.

21. The percutaneous absorption type medical preparation according to claim 20 **characterized in that** the organic acid is acetic acid.

22. The percutaneous absorption type medical preparation according to claim 1 **characterized in that** it is obtained dissolving or dispersing a mixture containing cyclodextrin and/or the cyclodextrin derivative with the drug in the nonaqueous base.

23. The percutaneous absorption type medical preparation according to claim 22 **characterized in that** the preparation is obtained by dissolving the mixture in a solvent, followed by evaporating the solvent to dryness.

24. The percutaneous absorption type medical preparation according to claim 22 **characterized in that** it is obtained by grinding the mixture in a dry or wet conditions in the same batch.

25. The percutaneous absorption type medical preparation according to claim 22 **characterized in that** the mixture further comprises a plasticizer.

26. The percutaneous absorption type medical preparation according to claim 22 **characterized in that** the mixture further comprises an absorption promoter.
